Europäisches Patentamt

European Patent Office

Office européen des brevets

(19)

(11) **EP 1 103 254 B1**

(12) **EUROPÄISCHE PATENTSCHRIFT**

(45) Veröffentlichungstag und Bekanntmachung des Hinweises auf die Patenterteilung:
**23.03.2005 Patentblatt 2005/12**

(51) Int Cl.⁷: **A61K 9/48**

(21) Anmeldenummer: **99811071.2**

(22) Anmeldetag: **19.11.1999**

(54) **Verfahren zum Herstellen eines Stärke enthaltenden Formkörpers, homogenisierte Stärke enthaltende Masse und Vorrichtung zum Herstellen einer Weichkapsel**

Process to manufacture starch-containing shaped bodies, mass containing homogenized starch and device to manufacture soft capsules

Procédé de préparation de corps moulés, masse à base d'amidon homogénéisé et dispositif pour préparer des capsules molles

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(43) Veröffentlichungstag der Anmeldung:
**30.05.2001 Patentblatt 2001/22**

(73) Patentinhaber: **Swiss Caps Rechte und Lizenzen AG**
**9533 Kirchberg (CH)**

(72) Erfinder:
• **Engel, W.Dieter**
**9524 Zuzwil (CH)**
• **Brocker, Erich, Dr.**
**9533 Kirchberg (CH)**
• **Ménard, Rico**
**8044 Zürich (CH)**
• **Tomka, Ivan, Dr.**
**8702 Zollikon (CH)**

(74) Vertreter: **Wenger, René et al**
**Hepp, Wenger & Ryffel AG**
**Friedtalweg 5**
**9500 Wil (CH)**

(56) Entgegenhaltungen:
EP-A- 0 304 401      WO-A-90/05161
WO-A-90/14938      WO-A-92/04408
WO-A-92/09274      WO-A-97/35537
GB-A- 2 190 093

**Beschreibung**

Die Erfindung betrifft ein Verfahren zum Herstellen eines Stärke enthaltenden Formkörpers, eine homogenisierte, Stärke enthaltende Masse und eine Vorrichtung zum Herstellen einer Weichkapsel gemäss der Oberbegriffe der unabhängigen Ansprüche.

Formkörper aus biologisch abbaubaren Materialien sind aus Gründen des Umweltschutzes schon seit längerem von ausserordentlichem Interesse. Als Folge der BSE-Problematik gewinnen insbesondere Kapseln mit einer Kapselhülle aus gelatinefreien Materialien für die Verabreichung pharmazeutisch wirksamer Substanzen, an Bedeutung.

In einer Reihe von Publikationen wird die Herstellung von Steckkapseln aus Stärke beschrieben, wie z.B. in EP 118 240 und US 4,738,724. Die Steckkapseln werden als zweiteilige Hülle im Spritzgussverfahren vorfabriziert und, gegebenenfalls nach Zwischenlagerung, mit hochviskosen oder festen Wirksubstanzen gefüllt. Aufgrund von Undichtigkeiten der Steckverbindung eignen sich Steckkapseln nicht für niedrigviskose Flüssigkeiten. Zudem ist der Herstellungsprozess einer gefüllten Steckkapsel aufwendig und kostspielig, da die Arbeitsschritte Herstellen und Füllen der Kapselhülle getrennt voneinander vorgenommen werden.

Für pumpbare, im weitesten Sinne flüssige Kapselinhaltsstoffe haben sich Kapseln mit einer einteiligen Kapselhülle aus Gelatine durchgesetzt, die in kontinuierlichen, automatisierbaren Verfahren hergestellt werden können. Die Herstellung der Kapselhülle und das Füllen derselben geschieht dabei in einem einzigen Arbeitsschritt. In diesen kontinuierlichen, 1-Schritt-Verfahren werden Formteile gefertigt, aus denen die Kapselhülle während und nach dem Füllen durch Verschweissen der Aussenkanten der Formteile zusammengefügt werden. Die Formteilfertigung geschieht entweder mittels auseinander- und zusammengehender Formen, wie z.B. im Norton-, Banner und Schering-Prozess oder mittels rotierender Formwalzen, wie es z.B. im Rotary-Die-Prozess und im Accogel-Verfahren verwirklicht ist ("Die Kapsel" Fahrig/Hofer-Herausgeber, Stuttgart, 1983; Lachmann/Liebermann/Kanig, "The Theory and Practice of Industrial Pharmacy"; Third Edition, Philadelphia 1986). Das Füllen erfolgt mit Hilfe von Dosierpumpen, die eine definierte Menge Wirksubstanz während des Ausstanzens und Verschweissens der Formteile zur Bildung einer einteiligen Kapselhülle abgeben. Das Verschweissen, d.h. die Ausbildung der Nähte erfolgt generell durch Druck und Wärme. Die Herstellkosten sind gegenüber der Herstellung von zweiteiligen Steckkapseln erheblich reduziert.

US 5,342,626 beschreibt die Herstellung von Kapseln im Rotary-Die-Prozess, wobei das Kapselhüllmaterial aus Carrageenan, Mannan-Gums, wie z.B. Galacto- und Glucomannanen, Gelan bzw. Mischungen untereinander besteht. Diese makromolekularen pflanzlichen Biopolymere sind jedoch aus Kostenüberlegungen inakzeptabel, da die Rohstoffe zu teuer sind.

Der Herstellungsprozess für einteilige Kapseln stellt an das Kapselhüllmaterial eine Reihe von Anforderungen. Eine der Hauptvoraussetzungen ist die Fähigkeit des Kapselhüllmaterials hochelastische "endlose" Bänder mit einer ausreichenden Festigkeit auszubilden. Die Kapselhülle muss sich bei Bedarf im Magendarmtrakt rasch lösen, um die Wirksubstanzen freisetzen zu können. Das Kapselhüllmaterial muss verschweissbar sein. Die Moleküle des die Formteile bildenden Materials, insbesondere die Makromoleküle des Polymeren, sollten sich an der Nahtstelle idealerweise durchdringen, um eine ausreichende Stabilität der Nahtstelle zu gewährleisten. Gelatine erfüllt all diese Bedingungen in nahezu idealer Weise und konnte als Material für einteilige Kapselhülle bislang nicht ersetzt werden.

Unter Verfügbarkeits- und Kostenkriterien ist Stärke auch für die Herstellung einteiliger Kapselhüllen ein wünschenswertes Ausgangsmaterial.

Die Herstellung von Stärkefilmen wurde schon mehrfach beschrieben, die Kombination von Eigenschaften, die ein solcher Stärkefilm zur Herstellung einteiliger Kapselhüllen aufweisen muss, wurde bis anhin nicht erreicht.

EP 474 705 beschreibt ein Verfahren zur Herstellung von Stärkeformkörpern durch Extrusion einer Stärkeschmelze. Die Stärkeschmelze enthält Stärke mit einem Amylosegehalt über 50 % und Zuschlagstoffen. Aus der Schmelze wird vor, während und/oder nach dem Extrudieren das Wasser durch Anlegen von Unterdruck entfernt. Die aus diesem Material extrudierten Folien weisen eine Bruchdehnung zwischen 80 und 200% auf. Hochamylosehaltige Stärken sind als Kapselhüllmaterial nicht geeignet, da die Tendenz der Amyloseketten zur Retrogradation einem schnellen Auflösen der Kapselhülle entgegensteht.

EP 0 397 819 offenbart ein Verfahren zum Herstellen thermoplastisch verarbeitbarer Stärke, wobei der kristalline Anteil in der Stärke unter 5% liegt. Das Verfahren besteht im Mischen nativer Stärke mit mindestens 10 Gew. % eines Zuschlagstoffes, welcher einen Löslichkeitsparameter von mindestens 30,7 $(MPa)^{1/2}$ besitzt. Die Mischung wird unter Wärmezufuhr in einem Temperaturbereich zwischen 120°C und 220°C in eine Schmelze überführt. Der Wassergehalt der Stärke wird bereits in der Schmelze auf unter 5% reduziert. Die Molmasse der eingesetzten Stärke ist vor der Überführung in den thermoplastischen Zustand grösser als 1 000 000 Dalton, bevorzugt zwischen 3 000 000 Dalton und 10 000 000 Dalton. Dieses Verfahren liefert zwar eine thermoplastische Stärke mit guter Verarbeitbarkeit zu Formkörpern, welche eine ausreichende Festigkeit aufweisen, die Bruchdehnung der mit dieser thermoplastischen Stärke hergestellten Formkörper erreicht jedoch nur Werte zwischen 40 und 55%. Die Elastizität der Stärkefilme ist damit für die Herstellung einteiliger Kapselhüllen in kontinuierlichen Verfahren zu gering und führt zu einem Reissen der Formteile bei der Herstellung bzw. zu Rissen in der fertigen Kapsel.

Der Stärkefilm, welcher nach dem in EP 397 819 offenbartem Verfahren erzeugt wird, zeigt ausserdem nicht die Verschweissbarkeit bzw. Nahtfestigkeit, die den Qualitätsanforderungen einteiliger Kapselhüllen genügen würde.

EP 304 401 beschreibt ebenfalls ein Verfahren zur Herstellung geformter Gegenstände aus Stärke. Die dazu notwendige thermoplastische Stärkeschmelze wird aus einer vorbehandelten Stärke hergestellt. Die Destrukturierung (Zerstörung der kristallinen Bereiche) der nativen Stärke und die anschliessende Homogenisierung (überführen in den thermoplastischen Zustand) findet jeweils bei Temperaturen zwischen 120°C und 190°C in einem geschlossenen Gefäss mit einem Wassergehalt zwischen 10 und 20% statt. Die Bruchdehnung der nach diesem Verfahren hergestellten Stärkefilme ist für die Produktion einteiliger Kapselhüllen in kontinuierlichen Verfahren nicht ausreichend. Die Stärkefilme zeigen darüberhinaus auch eine unzureichende Verschweissbarkeit und Nahtfestigkeit.

EP 0 542 155 offenbart biologisch abbaubare Formmassen, die sich unter anderem zur Filmherstellung eignen. Die Formmassen enthalten neben thermoplastisch verarbeitbaren Stärke Zellulosederivate. Die Bruchdehnung übersteigt den Wert von 85 % jedoch nicht, was für die Herstellung einteiliger Kapselhüllen in kontinuierlichen Verfahren nicht ausreicht. Die Verschweissbarkeit der Filme ist unbefriedigend. Viele der in EP 542 155 offenbarten Polymerblends enthalten Substanzen, die für pharmazeutische Anwendungen und für Nahrungsmittel nicht zugelassen sind.

WO 97/35537 offenbart mittels rotierender Formwalzen hergestellte gelierte Stärke enthaltende einteilige Kapseln. Die dort offenbarte Lehre des teilweisen Anlösens der Filmoberfläche hat sich für die Herstellung einteiliger Kapseln nachteilig hinsichtlich der Transport- und Druckstabilität (beim Herausdrücken der Kapseln aus den Blisterverpakkungen) erwiesen, da die Kapselhüllen im Bereich der Nahtstelle, dadurch zu weich und flexibel werden.

Die Aufgabe der vorliegenden Erfindung ist die Vermeidung der Nachteile des Standes der Technik.

Insbesondere besteht die Aufgabe der vorliegenden Erfindung darin, ein Verfahren zur Herstellung gelatinefreier Formkörper bereitzustellen.

Eine weitere Aufgabe besteht darin, eine Stärke enthaltende Mischung bereitzustellen, welche zu einteiligen Kapselhüllen mittels halbkontinuierlicher oder kontinuierlicher Verfahren, insbesondere mittels des Rotary-Die-Prozess, verarbeitet werden kann.

Eine weitere Aufgabe besteht darin, einteilige Weichkapseln auf Stärkebasis bereitzustellen, wobei der Stärkefilm zur Herstellung der Kapselhülle eine Bruchdehnung von mindestens 100% aufweisen soll.

Eine weitere Aufgabe besteht darin, Stärkefilme mit guter Verschweissbarkeit bereitzustellen.

Eine weitere Aufgabe besteht darin, Stärkekapseln mit einteiliger Kapselhülle in kontinuierlichen Verfahren herzustellen, welche nach einer Lagerdauer von einem Jahr weder Undichtigkeiten, insbesondere an der Nahtstelle, zeigen, noch Veränderungen der Auflösungsgeschwindigkeit der Kapselhülle.

Diese Aufgaben werden gelöst durch die Merkmale der unabhängigen Ansprüche.

Insbesondere werden sie gelöst durch ein Verfahren zum Herstellen eines Stärke enthaltenden Formkörpers, insbesondere einer Weichkapsel mit einteiliger Kapselhülle, wobei das Verfahren folgende Schritte umfasst

a Überführung einer Mischung enthaltend mindestens eine native oder chemisch-modifizierte Stärke mit einem Amylopektingehalt grösser oder gleich 50 Gew.-% bezogen auf das Gewicht der wasserfreien Stärke, Wasser, und mindestens einen organischen Weichmacher, wobei der Gehalt an organischem Weichmacher in einem Bereich von 37 Gew.-% bis 50 Gew.-% bezogen auf das Gewicht der wasserfreien Stärke liegt, unter Aufschmelzen und Kneten in eine homogenisierte thermoplastische Schmelzmasse in einer ersten Verarbeitungseinrichtung;

b gegebenenfalls Herstellen eines lagerfähigen Zwischenproduktes, insbesondere eines Granulates nach Abkühlen der homogenisierten Schmelzmasse und nachfolgendes Einschmelzen des Zwischenproduktes in einer zweiten Verarbeitungseinrichtung;

c Herstellen wenigstens eines Materialsstranges, insbesondere eines extrudierten Films, am Ausgang der ersten oder gegebenfalls zweiten Verarbeitungseinrichtung,

d Umformen des Materialstranges zu einem Formkörper in einem kontinuierlichen oder intermittierenden Formverfahren;

e gegebenenfalls Trocknen des Formkörper,

wobei die Schritte a) bis c) derart durchgeführt werden, dass in Schritt d) ein Wert des Staudinger-Index $[\eta]$ der den Materialstrang bildenden Masse von mindestens 40 ml/g, bevorzugt von mindestens 50 ml/g und noch bevorzugter von mindestens 60 ml/g resultiert.

Die in Schritte a) eingesetzte Mischung enthält die Stärke vorzugsweise in einem Gewichtsbereich von 45 bis 80 Gew.% bezogen auf das Gesamtgewicht der Mischung.

Der Begriff "einteilig" soll zur Abgrenzung gegenüber den zweiteiligen Kapseln verstanden werden, welche durch Stecken und/oder Verkleben zweier Kapselteile mit übereinandergelegten Aussenkanten erzeugt werden. Die einteilige Kapselhülle kann gänzlich ohne Nahtstelle oder aber, wenn aus Formteilen gebildet, mit verschweisster Nahtstelle, ausgebildet sein.

In einer bevorzugten Ausführungsform enthält die in Schritt a) eingesetzte Mischung zusätzlich ein internes Gleit- und Formtrennmittel, welches ausgewählt ist aus der Gruppe bestehend aus Lecithinen, Mono-, Di- oder Triglyceriden von Speisefettsäuren, Polyglycerinester der Speisefettsäuren, Polyethylenglycolester der Speisefettsäuren, Zuckerester der Speisefettsäuren und Speisefettsäuren.

Das Gleit- und Formtrennmittel ist in der Mischung bevorzugt in einem Bereich von 0 bis 4 Gew.% bezogen auf das Gesamtgewicht der Mischung enthalten. Vorzugsweise wird es der Mischung in 0,5 bis 2 Gew.% und noch bevorzugter in 0,8 bis 1,5 Gew.% zugesetzt.

In einer bevorzugten Ausführungsform enthält die in Schritt a) eingesetzte Mischung , Glycerinmonostearat und Lecithin in einem Gewichtsverhältnis von 1 : 1,5, bevorzugt von 1 : 1,2 und noch bevorzugter von 1:1.0 Glycerinmonostearat und Lecithin wirken sich in diesem Gewichtsverhältnis sehr vorteilhaft sowohl auf die Verschweissbarkeit als auch auf die Schweissnahtfestigkeit aus.

Unter Speisefettsäuren werden die als Säurekomponenten der Triglyceride natürlicher Fette vorkommenden Monocarbonsäuren verstanden. Sie weisen eine gerade Anzahl von C-Atomen auf und haben ein unverzweigtes Kohlenstoffgerüst. Die Kettenlänge der Fettsäuren variiert von 2 bis 26 C-Atomen. Eine grosse Gruppe der Fettsäuren sind gesättigte Fettsäuren.

Der Begriff Weichkapsel ist als Produkt der in der Literatur aufgeführten gängigen kontinuierlichen und halbkontinuierlichen, 1-Schritt-Herstellungsverfahren für einteilige Kapseln zu verstehen. Er dient dabei weniger zur Differenzierung des Weichmachergehaltes, da auch Hartkapseln, als Bezeichnung für zusammengefügte zweiteilige Kapseln, einen Weichmachergehalt von bis zu 12% bezogen auf die Gesamtmasse enthalten können.

Unter dem Begriff Stärke sollen native Stärken, sowie physikalisch und/oder chemisch modifizierte Stärken verstanden werden. Für die in Schritt a) des erfindungsgemässen Verfahrens eingesetzte Mischung sind alle Stärken, deren Amylopektingehalt über 50% bezogen auf das Gesamtgewicht der wasserfreien Stärke liegt, unabhängig von der Pflanze, aus der sie gewonnen werden, geeignet. Als für das (hier beschriebene)Verfahren besonders geeignet hat sich Kartoffelstärke erwiesen.

In einer weiteren bevorzugten Ausführungsform handelt es sich bei der Stärke um vorverkleisterte Stärke. Oberhalb einer für jede Stärkeart typischen Temperatur tritt in wässrigen Stärkesuspensionen nach Erreichen eines Höchstquellungsgrades "Lösung", d.h. irrevesible Desintegration der Moleküle, ein. Dieser Vorgang wird auch als "Verkleisterung" bezeichnet. Die Verkleisterung, d.h. die irreversible Quellung bei höherer Temperatur bis zum 40-fachen des ursprünglichen Volumens beruht auf einer allmählichen Wasseraufnahme und Lösung von Wasserstoffbrücken-Bindungen, die eine weitere Hydratation bis zur völligen Desintegration des Stärkekorn-Gefüges ermöglicht.

Die Überführung der Stärke enthaltenden Mischung in den thermoplastischen, homogenisierten Zustand in Schritt a), ebenso wie die danach folgenden Verarbeitungsschritte b) und c) , müssen unter Bedingungen erfolgen, die einen unkontrollierten Abbau der Amylose- und Amylopektinmasse zu kurzen Bruchstücken verhindern.

Die Verarbeitungsparameter wie z.B. Temperatur, Druck, Verweilzeit und Knetleistung, während der Schritte a) bis c) müssen derart zusammenwirken, dass der Staudinger-Index $[\eta]$ der den Materialstrang bildenden homogenisierten Masse im Schritt d) mindestens 40 ml/g beträgt. In einer bevorzugten Ausführungsform beträgt der Staudinger-Index $[\eta]$ mindestens 50 ml/g und noch bevorzugter mindestens 60 ml/g.

Durch die Schritte a) bis c) muss eine Masse erzeugt werden, in der im wesentlichen keine kristallinen Bereiche in der Stärke mehr vorhanden sind. Kristalline Bereiche führen im extrudierten Materialstrang zu Stippenbildung, d.h. zu Inhomogenitäten im Material, die sich besonders dann nachteilig auswirken, wenn der Materialstrang in Schritt c) ein extrudierter Film ist. Mit "im wesentlichen keine kristallinen Bereiche" soll gemeint sein, dass diese soweit zerstört sind, dass eine Beeinträchtigung der bezüglich der Umformung relevanten physikalischen Parameter des extrudierten Materials nicht auf das Vorhandensein kristalliner Bereiche zurückgeführt werden kann.

Unter dem Begriff "homogen" bzw. "homogenisiert" soll somit ein Material oder eine Masse verstanden werden, die an jeder Stelle im Material die im wesentlichen gleiche chemische und physikalische Zusammensetzung und Beschaffenheit aufweist. Zu geringfügigen Abweichungen kann es an den jeweiligen Material- oder Formteiloberflächen durch Aufnahme von Luftfeuchtigkeit kommen.

Der Staudinger-Index $[\eta]$ oder auch Grenzviskosität steht innerhalb einer polymerhomologen Reihe mit der Molmasse dem Gewichtsmittel der Molekulargewichtsverteilung in folgendem Zusammenhang

$$[\eta] = K \times M^{\alpha}.$$

wobei $\alpha$ ein von der Molekül-Gestalt abhängiger Exponent und der K-Wert eine von der gelösten Substanz und vom Lösungsmittel abhängige Konstante ist. Der StaudingerIndex ist innerhalb der polymerhomologen Reihe umso grösser, je grösser das Molekulargewicht des Polymeren bei ansonsten unveränderten Parametern ist. Eine Ermittlung der absoluten Molekulargewichte kann die Messung des Staudinger-Index nicht leisten.

Ohne eine erschöpfende Erklärung zu liefern, wird vermutet, dass in erster Linie der Polymerisationsgrad der verzweigten Amylopektinemoleküle der eingesetzten Stärke sich für die Elastizität des in Schritt d) erzeugten Materialstranges verantwortlich zeigt. Der Polymerisationsgrad bzw. das Molekulargewicht des Amylopektins sollte genügend hoch sein. Dies ist insbesondere für einen bahnförmigen Film der im 1-Schritt Verfahren zu einer Weichkapsel geformt wird, von grosser Bedeutung.

Zusätzlich zur inherenten Elastizität der Amylopektinmoleküle bei genügendem Polymerisationsgrad, kann auch eine Art "Stärke-Netzwerk" durch Verschlingung und Verhakung durch die Verzweigungen der Amylopektinmoleküle aufgebaut werden: Aber auch Amylosemoleküle können bei genügend hohem Polymerisationsgrad, an diesem "Stärke-Netzwerk" partizipieren.

Der Umformungsvorgang des Materialstranges zu einem Formkörper, insbesondere die Umformung eines extrudierten Films in eine einteilige Weichkapsel mit den in der Technik bekannten Verfahren, erfordert Bruchdehnungen des Materialstranges, insbesondere des Films von mindestens 100% bei 25°C und 60% relativer Luftfeuchtigkeit. In einer bevorzugten Ausführungsform ist die Bruchdehnung des Materialstranges, insbesondere Films, mindestens 160% und noch bevorzugter mindestens 240%.

Bruchdehnungen von 100% können mit der nach dem erfindungsgemässen Verfahren hergestellten stärkehaltigen Masse erreicht werden, wenn der Staudinger-Index der Masse mindestens 40 ml/g beträgt, bevorzugt mindestens 50 ml/g und noch bevorzugter mindestens 60 ml/g.

Neben der Bruchdehnung spielen die maximale Festigkeit $\sigma_m$ des Stanges für die Eigenschaften der daraus hergestellten Formkörper, eine wesentliche Rolle. Die Festigkeit ist von besonderer Bedeutung, wenn es sich bei dem Materialstrang um einen Film handelt mit dem im Umformungsprozess eine einteilige Kapselhülle gebildet wird. Die Festigkeit $\sigma_m$ des in Schritt c) erzeugten Materialstranges, insbesondere Films, muss bei 25°C und 60% relativer Luftfeuchtigkeit wenigstens 2 MPa betragen. In einer bevorzugten Ausführungsform in einem Bereich von 3,5 MPa bis 8 MPa und noch bevorzugter in einem Bereich von 4MPa bis 6,5 MPa liegen.

Auch die Festigkeit steht in Zusammenhang mit dem Staudinger-Index $[\eta]$. Es wurde gefunden, dass $\sigma_m$ des in Schritt c) resultierenden Materialstranges umso grösser ist, je grösser der Staudinger-Index der den Materialstrang bildenden Masse ist.

Die chemische Substitution der Stärkehydroxylgruppen unter Ether-, Esther -, Vinyl- und Acetalbildungen können vorteilhaft sein, da sie die Ausbildung der "Stärke-Netzwerke" fördern. Vorteilhaft sind insbesondere Hydroxypropyl-Stärkederivate. Ein Hydroxypropyl-Stärkederivat mit DS = 0,1 (DS = Degree of Substitution) zeigt neben sehr guten Filmbildungseigenschaften auch die Erfüllung der oben genannten Parameter.

Da Schritt d) und Schritt e) unter Bedingungen erfolgen, die keinen weiteren Abbau der Amylose- und Amylopektinmoleküle bewirken, liegen die Moleküle bezüglich ihres Polymerisationsgrades (und damit Grenzviskosität der die Formteile bildenden Masse) auch im resultierenden Formkörper und insbesondere in der Kapselhülle der Weichkapsel, unverändert vor.

Die Auswirkung der verschiedenen Verarbeitungsparameter auf den Abbau der Stärkemoleküle und somit auf die Entwicklung des Staudinger-Index sind dem Fachmann bekannt. So kann z.B. auch bei relativ hohen Temperaturen ein weitgehender Abbau der Stärkemoleküle vermieden werden, wenn die Verweilzeiten der Stärke enthaltenden Masse bei diesen Temperaturen klein gehalten wird.

In einer bevorzugten Ausführungsform übersteigt die Temperatur der Schmelzmasse in der ersten und gegebenfalls zweiten Verarbeitungseinrichtung , sowie beim Herstellen des Materialstranges 160°C , bevorzugt 120°C und noch bevorzugter 90°C nicht. Bei 160°C sollte insbesondere auch der Aufschlussvorgang in Schritt a) in weniger als 5 Minuten, bevorzugt weniger als 3 Minuten abgeschlossen sein.

In einer weiteren bevorzugten Ausführungsform überschreitet die durch das Kneten eingebrachte Energie bei der Erzeugung der homogenisierten thermoplastischen Schmelzmasse in Schritt a) bis c) 0,3 kWh/kg, bevorzugt 0,2 kWh/kg und noch bevorzugter 0,175 kWh/kg nicht.

Der Weichmachergehalt der in Schritt a) eingesetzten Mischung ist in einem Bereich von 30 Gew.% bis 50 Gew.% und noch bevorzugter in einem Bereich von 38 Gew.% bis 45 Gew.%. bezogen auf das Gewicht der wasserfreien Stärke.

Durch den durch die Prozessführung bewirkten weitgehenden Ausschluss stark abgebauter Oligomere der Stärke gelingt es, die hohen Mengen an Weichmachern wie sie in den bevorzugten Ausführungsbeispielen vorgesehen sind, in die homogenisierte Masse einzuarbeiten. Oligomere wie z.B. Maltodextrine, würden in der thermoplastischen Schmelze bzw. im daraus resultierenden Produkt ebenfalls weichmachende Wirkung entfalten und die Einarbeitung zusätzlich grosser Mengen bevorzugter "externer Weichmacher" wäre nicht mehr möglich.

Bevorzugt werden solche Weichmacher eingesetzt, die einen Löslichkeitsparameter von gleich oder > 16,3 $(MPa)^{1/2}$ aufweisen. Die Weichmacher werden ausgewählt aus der Gruppe bestehend aus Polyalkoholen, organischen Säuren, Aminen, Säureamiden und Sulfoxiden. Bevorzugt sind Polyalkohole. Als der geeignetste Weichmacher hat sich Glycerin erwiesen.

Überraschenderweise hat sich gezeigt, dass der Gehalt an Glycerin in der in Schritt a) eingesetzten Mischung in bestimmten Gewichtsbereichen durch Wasser ersetzt werden kann, ohne dass es zu einer Verschlechterung der Eigenschaften des in Schritt d) erzeugten Formkörpers führt. Insbesondere die Bruchdehnung wird bei einem Ersatz des Glycerins durch Wasser im Verhältnis 2:1 (2 Teile Glycerin werden ersetzt durch ein Teil Wasser) nicht herabgesetzt. Der Glycerin-Gehalt beträgt jedoch mindestens 12 Gew.% bezogen auf das Gewicht der wasserfreien Stärke.

Der in Schritt a) eingesetzten Mischung kann je nach erforderlichen Eigenschaften des in d) und e) resultierenden Formkörpers noch mindestens ein Zuschlagstoff in einem Gewichtsbereich von 3,5 Gew.% bis 15 Gew.%, bevorzugt von 5 Gew.% bis 8 Gew.% bezogen auf das Gesamtgewicht der Mischung zugesetzt werden. Die Zuschlagstoffe werden ausgewählt aus der Gruppe bestehend aus Carbonaten und Hydrogencarbonaten der Alkali- und Erdalkaliionen, weitere Zerfallshilfen, Füllstoffe, Farbstoffe, Antioxidantien, physikalisch und/oder chemisch modifizierte Biopolymere insbesondere Polysaccharide und pflanzliche Polypeptide.

Opazität der homogenisierten Masse wird z.B. bevorzugt mit dem Zusatz von Titaniumdioxid als Füllstoff erreicht.

Als Zerfallshilfe, für einen schnellen Zerfall der Kapselhülle werden bevorzugt Calciumcarbonat und Amylasen zugesetzt.

Die Gruppe der physikalisch und/oder chemisch modifizierten Biopolymere umfasst Cellulose, insbesondere teilhydroxypropylierte Cellulose, Alginate, Carageenan, Galactomannane, Glucomannane, Casein.

Die homogenisierte Stärkeschmelze kann im Schritt c) direkt mittels Breitschlitzdüse zu einem Stärkefilm bzw. Stärkeband extrudiert werden. Die Schmelzmasse kann jedoch auch abgekühlt, getrocknet und zu einem lagerfähigen (unter Feuchtigkeitsabschluss) Granulat verarbeitet werden. Dieses Granulat kann gelagert werden und steht für eine spätere Verarbeitung zur Verfügung. Optional können der zu Granulaten verabeiteten Schmelze auch nur ein Anteil der notwendigen Gleit- bzw. Formtrennmittel, Weichmacher und Zuschlagstoffe zugefügt werden. Es kann z.B. auf die Zugabe der tierischen und/oder pflanzlichen Fette zur Vermeidung unerwünschter Farbeffekte verzichtet werden und diese erst beim Wiederaufschmelzen des Granulates in der zweiten Verarbeitungseinrichtung zugemischt werden.

Die extrudierten Bänder werden nun entweder direkt weiterverarbeitet oder gegebenenfalls zur Lagerung unter Verwendung von Kunststofffolien als Zwischenschicht auf Rollen aufgewickelt. Als geeignetstes Folienmaterial hat sich dabei Polyethylen erwiesen.

Der erfindungsgemässe Stärkefilm kann insbesondere für Herstellung von Weichkapseln auf allen in der Technik bekannten Anlagen zur Herstellung einteiliger Kapseln verarbeitet werden. Als besonders geeignet haben sich kontinuierliche Anlagen und insbesondere der Rotary-Die-Prozess erwiesen. Die Kapselwand wird dabei aus zwei vorab aus einem Stärkefilm herausgestanzten Formteilhälften unter Wärmewirkung verschweisst. Zwei "endlose Stärkefilme" werden durch zwei benachbarte, in gegenläufigem Sinn rotierenden Rollen oder Walzen mit Aussparungen geführt. Während der Stärkefilm in die Aussparung gepresst und somit die Kapselhälften geformt werden, wird die pump- und spritzbare Kapselfüllung mittels eines Ventils exakt dosiert und über einen Füllkeil in den Einzugswinkel der Formwalzen eingebracht. Die Form und Grösse der Kapsel ist somit abhängig von den geometischen Abmessungen der Aussparungen in den Walzen und dem eindosierten Füllvolumen.

Konsequenterweise soll unter dem Begriff Kapsel deshalb nicht nur die typischen Kapselformen verstanden werden, sondern auch jede andere mögliche Form von "Hüllen", wie z.B. Kugeln, Kissen und Figuren. Bis heute existieren zahlreiche Weiterentwicklungen und Abweichungen von diesem grundlegenden Prinzip.

Die mittels des erfindungsgemässen Stärkefilms hergestellten einteiligen Kapselhüllen können zusätzlich beschichtet werden, z.B. um die Freisetzung von Wirksubstanzen zu verzögern.

Die Coextrusion, Beschichtung und das Laminieren des erfindungsgemässen Stärkefilms mit Materialien, deren filmbildende Eigenschaft auf synthetischen und/oder natürlichen Polymeren beruht, verschafft zusätzlich Möglichkeiten bestimmte Eigenschaften der Kapselhülle durch eine Mehrschichtfolie zu gestalten.

Insbesondere lässt sich durch den mehrschichtigen Aufbau eine Stärkefolie herstellen, die auf der Innenseite eine gut schweissbare Beschichtung aufweist, während die Aussenseite derart beschichtet wird, dass eine Retardwirkung des Zerfalls der Kapsel eintritt.

Der Wasseranteil der in Schritt a) eingesetzten Mischung kann im erfindungsgemässen Verfahren in Schritt b) oder c) gezielt verändert werden.

Teil der vorliegenden Erfindung ist daher weiterhin eine homogenisierte Stärke enthaltende Masse, welche mindestens eine im wesentlichen amorphe Stärke vorzugsweise in einem Gewichtsbereich von 45 bis 80 bezogen auf das Gesamtgewichte der Masse mit einem Amylopektingehalt von grösser oder gleich 50 Gew.% bezogen auf das Gewicht der wasserfreien Stärke enthält, weiterhin Wasser, mindestens einen organischen Weichmacher in einem Anteil von mindesten 12 Gew.% bezogen auf das Gewicht der wasserfreien Stärke, wobei der Staudinger-Index der homogenisierten Masse mindestens 40 ml/g beträgt.

Bevorzugt ist der Staudinger-Index mindestens 50 ml/g, noch bevorzugter mindestens 60 ml/g.

In einer bevorzugten Ausführungsform liegt der Gehalt an organischen Weichmachern im Bereich von 30 Gew.% bis 50 Gew.%, bevorzugt in einem Bereich von 38 Gew.% bis 45 Gew.%.

In einer weiteren Ausführungsform enthält die Masse zusätzlich ein Gleit- und Entformmittel, welches ausgewählt ist aus der Gruppe bestehend aus Licithinen Mono-, Di-, und Triglyceriden von Speisefettsäuren, Zuckerester der Speisefettsäuren und Speisefettsäuren.

Der Weichmacher wird vorzugsweise ausgewählt aus der Gruppe bestehend aus Polyalkoholen, organischen Säuren, Hydroxysäuren, Aminen, Säureamiden und Sulfoxiden. In einer bevorzugten Ausführungsform wird als Weichmacher Glycerin eingesetzt.

In einer weiteren Ausführungsform kann die Masse zusätzlich noch mindestens einen Zuschlagstoff in einem Gewichtsbereich von 3,5 Gew.% bis 15 Gew.% bezogen auf das Gesamtgewicht der Masse enthalten, bevorzugt von 5 Gew.% bis 8 Gew.%. Der Zuschlagstoff richtet sich nach den erforderlichen Eigenschaften des aus der homogenisierten Masse erzeugten Formkörpers und wird ausgewählt aus der Gruppe bestehend aus Carbonaten und/oder Hydrogencarbonaten der Alkali- und/oder Erdalkaliionen, weiteren Zerfallshilfen, Farbstoffen, Konservierungsmittel, Antioxidantien, physikalisch und/oder chemisch modifizierten Biopolymeren, insbesondere Polysaccharide und pflanzlichen Polypeptiden. Bevorzugt werden als Zerfallsmittel für einteilige Kapselhülle Kalziumcarbonat und Amylasen eingesetzt.

Der Formkörper, insbesondere die Kapselhülle hat eine Bruchdehnung von mindestens 100%, bevorzugt mindestens 160% und noch bevorzugter mindestens 240%.

Der Formkörper, insbesondere die Weichkapselhülle hat bei 25°C und 60% relativer Luftfeuchtigkeit eine Festigkeit von $\sigma_m$ von mindestens 2MPa, bevorzugt eine Festigkeit im Bereich von 3,5MPa bis 8MPa und noch bevorzugter von 4MPa bis 6,5MPa.

Teil der Erfindung sind weiterhin Formkörper, welche aus der erfindungsgemässen Masse hergestellt werden. Vorzugsweise ist der Formkörper eine einteilige Kapselhülle.

Der Formkörper, insbesondere die Kapselhülle hat eine Dicke im Bereich zwischen 0,1 und 2 mm bevorzugt zwischen 0,2 und 0,6 mm.

In einer weiteren bevorzugten Ausführungsform besteht der Formkörper, insbesondere die Weichkapselhülle aus einem mehrlagigen Film. Mindestens zwei der Filme haben eine unterschiedliche chemische Zusammensetzung.

Abgesehen von der Herstellung einschichtiger Kapselhüllen kann die thermoplastisch verarbeitbare Stärkeschmelze auch zur Herstellung jeglicher anderer Art von Formkörper insbesondere Verpackungsmaterialien verwendet werden.

In einer besonderen Ausführungsform enthält der Formkörper einen Wassergehalt von maximal 15 Gew.% bevorzugt maximal 7 Gew.% und noch bevorzugter maximal 5 Gew.% bezogen auf das Gesamtgewicht der Masse.

Zur Film- bzw. Bandführung werden keinerlei Schmierstoffe benötigt. Auch die Verschweissung der Kapselhälften erfolgt ohne Zusatz von Lösungsmitteln auf der Filmoberfläche, die einen anfängliche Verklebung der zusammengefügten Formteile dienen würden.

Ein Ausführungsbeispiel der Erfindung in vorrichtungsmässiger Hinsicht ist in den Figuren dargestellt und wird nachstehend genauer beschrieben. Es zeigen:

Figur 1      die Bruchdehnung [$\varepsilon_b$] der erfindungsgemässen Stärke enthaltenden Masse in Abhängigkeit des Staudinger-Index [$\eta$],

Figur 2      die maximale Festigkeit [$\sigma_m$] der erfindungsgemässen Stärke enthaltenden Masse in Abhängigkeit vom Staudinger-Index [$\eta$],

Figur 3      eine stark schematisierte Darstellung einer Füll- und Formstation im Rotary-Die-Verfahren, und

Figur 4      die symbolische Darstellung eines Doppel-Schneckenextruders mit den darin herrschenden Temperaturverhältnissen.

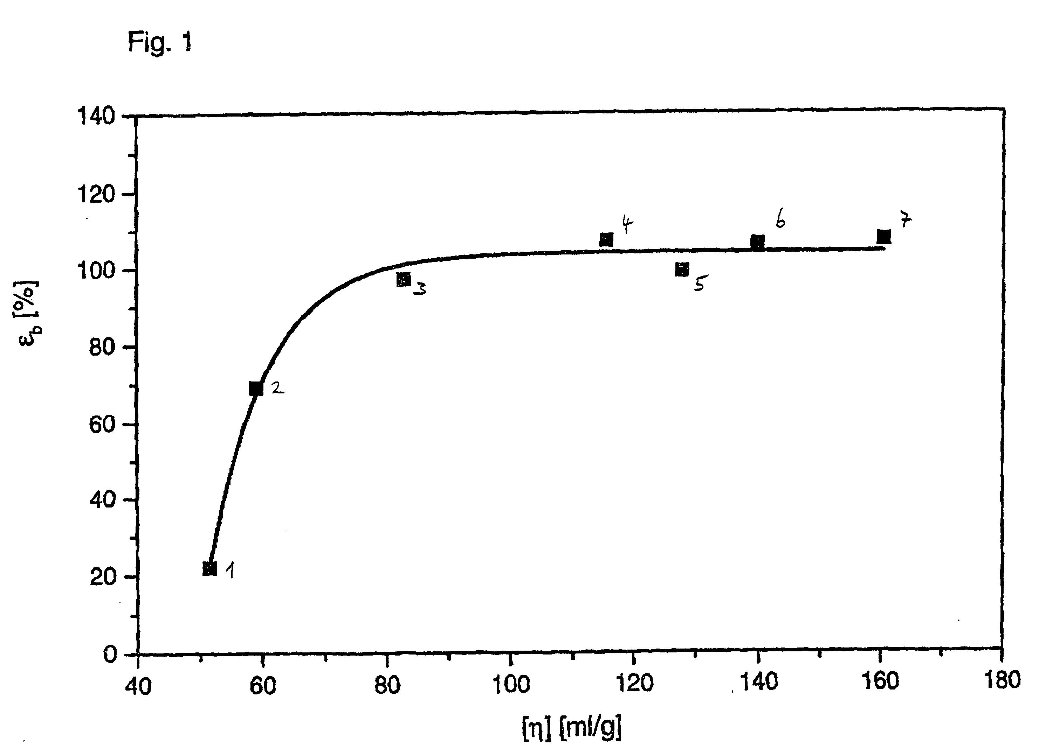

Die Herstellung der Proben, welche in Figur 1 den Zusammenhang zwischen Bruchdehnung und Staudinger-Index demonstrieren erfolgt folgendemassen:

| | |
|---|---|
| Stärke | 56,2 bis 56,9 Gew.% |
| Glycerin | 41,8 Gew.% bezogen auf den Gehalt der wasserfreien Stärke |
| Wasser | 1,3 - 2,0 Gew.% bezogen auf das Gesamtgew. der Mischung |

Die Messung der Bruchdehnung erfolgt nach DIN-Norm 53455 bzw. DIN EN ISO 527-1 bis 527-3. Die Mischungen wurden im Brabender-Kneter bei 160rpm bei einer Knetzeit von jeweils 15 min und bei Knettemperaturen von 110°C, 160°C, 200°C, 220°C und 235°C homogenisiert. Mit zunehmender Tepmperatur wurde infolge des thermischen Abbaus eine deutliche Braunfärbung festgestellt.

Die Messung des Staudinger-Index [$\eta$] erfolgt analog der DIN-Norm: DIN 51562-1 bis 51562-4. Allerdings musste nun der Glyceringehalt der Proben und sein Einfluss auf die Durchlaufzeiten im Ubbelohde-Viskosimeter berücksichtigt werden. Hierzu wurde zuerst der Einfluss des Glyceringehaltes auf die Durchflusszeit $t_0$ bestimmt, mittels der erhaltenen Eichgeraden konnten dann die Durchflusszeiten $t_{0Gly}$ bei einem beliebigen Glyceringehalt entsprechend

$$t_{0Gly} = t_0 \cdot (1{,}00002 + 0{,}00238 \cdot c_{Gly})$$

berechnet werden, wobei $c_{Gly}$ die vorliegende Konzentration Glycerin in mg/ml ist. Die für die abgebauten Stärken bestimmten Staudinger-Index sind zusammen mit den mechanischen Eigenschaften der zugehörigen Proben in der Tabelle 1 aufgeführt.

Die in Figur 1 gezeigte Abhängigkeit der Bruchdehnung der erfindungsgemässen Stärke enthaltenden Masse vom Staudinger-Index der Masse zeigt einen Anstieg der Bruchdehnung bei einem Wert des Staudinger-Index von 51,5 ml/g. Die Bruchdehnung steigt bis zu einem Wert von 97%.

97% Bruchdehnung wird bei einem Staudinger-Index von 82,8 ml/g erreicht. Danach läuft die Bruchdehnung mit zunehmendem Wert des Staudinger-Index asymptotisch einem Grenzwert von ca. 105% zu.

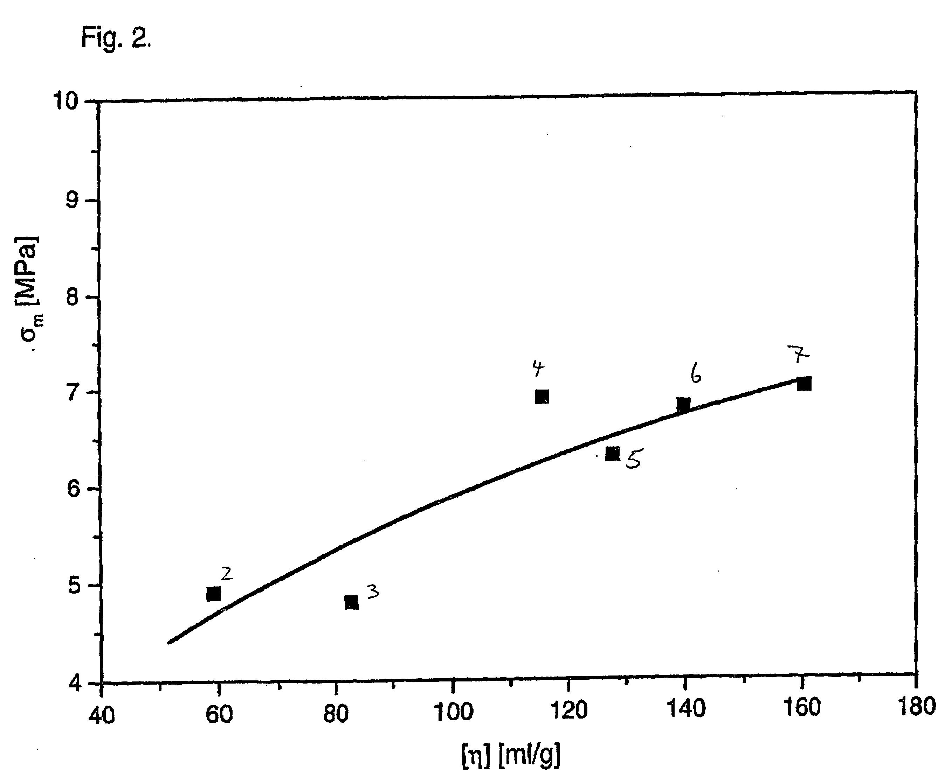

Bei geringerem Anteil des Weichmacheranteils verläuft die Kurve insgesamt flacher, d.h. zu tieferen Bruchdehnungen verschoben. Der Anfangswert des Staudinger-Index, d.h..der Wert ab dem ein merklicher Anstieg der Bruchdehnung beobachtet wird, ist unabhängig vom Weichmacheranteil und einzig abhängig vom Molekulargewichtsmittel der Stärkemoleküle bzw. den entsprechenden Staudinger-Index. Tabelle 1 führt die in Figuren 1 und 2 graphisch dargestellten Werte auf.

Die maximale Festigkeit $\sigma_m$ wurde analog DIN-Norm 53455 bzw. DIN EN ISO 527-1 bis ISO527-3 bestimmt. Für die in Figur 2 gezeigte Abhängigkeit wurde das gleiche Probenmaterial, für das in Figur 1 die Bruchdehnung gemessen wurde, verwendet.

Der Zusammenhang zwischen maximaler Festigkeit $\sigma_m$ und Staudinger-Index $[\eta]$ ist aus Figur 2 ersichtlich, wo eine Abnahme der Festigkeit mit kleiner werdendem StaudingerIndex festgestellt werden kann.

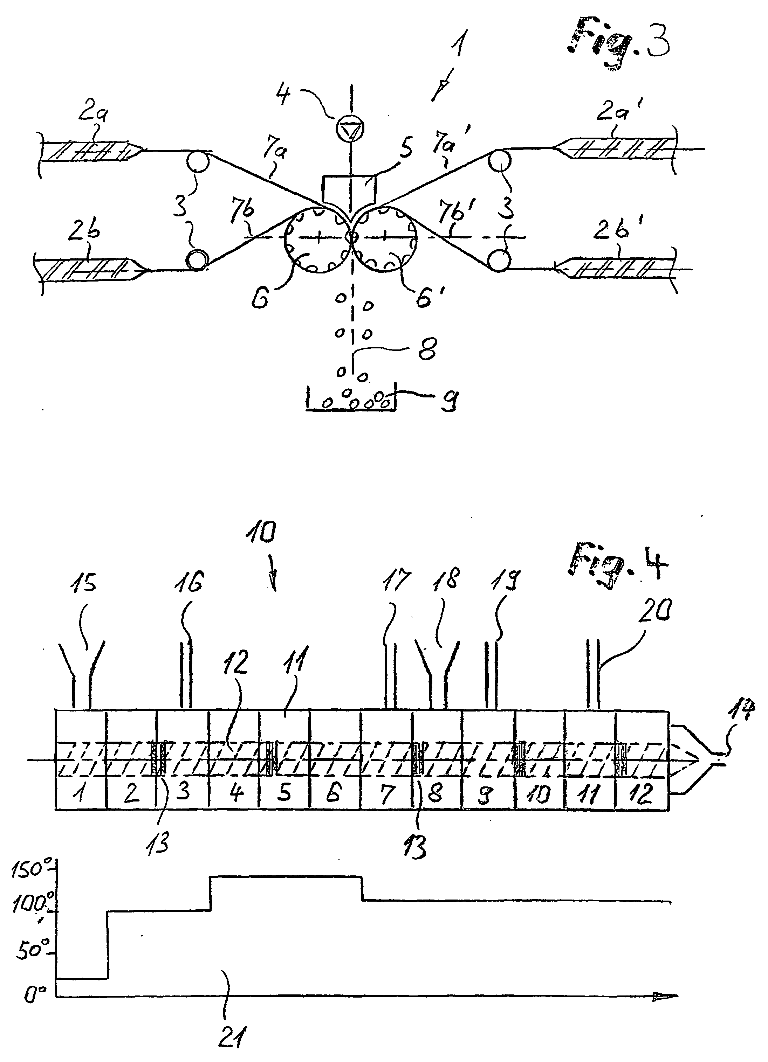

Die insgesamt mit 1 bezeichnete Füll- und Formstation in Figur 3 weist für die Verkapselung auf an sich bekannte Weise ein Formwalzenpaar 6, 6' auf, wobei in den Oberflächen der Formwalzen, die zur Formung der Kapseln erforderlichen Ausnehmungen angeordnet sind. Im Einzugszwickel des Formwalzenpaars ist ein Füllkeil 5 angeordnet, durch den mittels einer Förderpumpe 4 das Füllgut eingebracht werden kann. Beim vorliegenden Ausführungsbeispiel besteht die Kapselhülle aus zwei Schichten mit verschiedenen Materialeigenschaften, welche durch die beiden Stärkefilme 7a, 7a' einerseits und 7b, 7b' andererseits gebildet werden. Diese beiden Stärkefilme werden in den Schneckenextrudem 2a, 2a' und 2b, 2b' aufbereitet und über Umlenkwalzen 3 unmittelbar und mit gleicher Fördergeschwindigkeit dem Einzugszwickel des Formwalzenpaars 6, 6' zugeführt. Die Schneckenextruder sind dabei unmittelbar neben der Füll- und Formstation und gegebenenfalls auf dem gleichen Maschinengestell angeordnet.

Die Stärkefilme werden zwischen dem Formwalzenpaar zu einer einteiligen Weichkapsel geformt und verschweisst, wobei sie das Füllgut einschliessen. Die einzelnen Kapseln 9 werden aufgefangen und allenfalls einem Trocknungsprozess zugeführt, während das verbleibende Filmskelett 8 evtl. durch Recyling wieder zu neuen Kapseln verarbeitet werden kann.

Figur 4 zeigt stark vereinfacht einen Doppelschneckenextruder 10, der im vorliegenden Fall aus zwölf einzelnen Gehäuseblöcken 1 - 12 zusammengesetzt ist. Die Gehäuseblöcke sind von links nach rechts durchgehend nummeriert. Jeder Gehäuseblock lässt sich mit einem separaten Regelkreis elektrisch heizen und/oder mit ventilgesteuerten Zuflüssen mit Kaltwasser kühlen. Ausserdem können einzelne Blöcke mit Anschlussstutzen versehen sein, wie nachstehend noch erläutert wird. Im vorliegenden Fall handelt es sich um einen gleichdrehenden, engkämmenden Doppelschneckenextruder, wobei der Durchmesser einer Schnecke 44 mm beträgt. Die Länge der gesamten Schneckenwelle beträgt 2'112 mm, was einem Verhältnis von Länge zu Durchmesser von 48 entspricht. Am Ende des Extruders wird das Material über eine Düse 14 ausgetragen. Diese Düse kann beispielsweise zwölf Lochbohrungen von 2 mm Durchmesser aufweisen. Es wäre dabei denkbar, für die Granulatherstellung die einzelnen Materialstränge heiss abzuschlagen und dann einem Fluidbetttrockner zuzuführen. An der Düse 14 könnte aber auch unmittelbar ein fertiger Materialfilm abgezogen werden.

An den Schnecken 12 sind an geeigneten Stellen Knetscheiben 13 von unterschiedlicher Konfiguration angeordnet, um eine möglichst homogene Knetung der Materialmischung zu erreichen. Der Block 1 ist wassergekühlt und mit einem Pulvereinzug 15 versehen. Der Block 2 ist geschlossen während am Block 3 eine Einspritzdüse 16 für eine Flüssigdosierung in den Knetraum angeordnet ist. Im Übergangsbereich der Blöcke 2 und 3 sind feine neutrale Knetscheiben 13 angeordnet. Die Blöcke 4 bis 6 sind wiederum geschlossen, wobei an Block 5 breite, neutrale und rückfördernde Knetscheiben vorgesehen sind. Block 7 verfügt über eine Anschlussleitung 17, die mit einer Unterdruckquelle verbunden ist. An Block 8 ist wiederum ein Pulvereinzug 18 angeordnet und die Schnecke mit feinen, neutralen und oder fördernden Knetscheiben versehen. Block 9 verfügt ebenfalls über eine Einspritzdüse 19, während Block 10 geschlossen ist. Die Schnecke im Block 10 verfügt dagegen über breite, neutrale und rückfördernde Knetscheiben.

Block 11 hat eine weitere Abzugleitung 20, die mit einer Unterdruckquelle oder mit der Atmosphäre verbunden sein kann. Block 12 ist geschlossen, die Schnecke dort verfügt jedoch über mittlere, fördernde Knetscheiben.

Unterhalb der schematischen Förderschnecke ist eine Temperaturkurve aufgezeichnet. Die einstellbare Temperaturgenauigkeit beträgt +/- 3°C. Bei den angegebenen Temperaturen handelt es sich um die Blocktemperatur, die nicht zwingend mit der Temperatur in der Schmelze identisch sein muss. Die Temperatur in der Schmelze wird ersichtlicherweise noch durch andere Parameter beeinflusst, insbesondere durch die Drehzahl der Schnecke. Bei der Extrusion ist es deshalb erforderlich, diesen Gegebenheiten Rechnung zu tragen und die verstellbaren Grössen derart aufeinander abzustimmen, dass optimale Materialeigenschaften erzielt werden.

Bei dem anhand dieser Figur beschriebenen Ausführungsbeispiel wird eine Drehzahl von 340 Umdrehungen pro Minute (rpm) gefahren. Der gesamte Durchsatz beträgt ca. 34,3 kg/h und die Energieaufnahme beträgt ca. 0,175 kWh/kg. An dem auf 20°C gehaltenen Block 1 wird 20 kg/h (ca. 60%) Stärkepulver zudosiert. Das Pulver wird mit Schubkanten eingezogen und den auf 100°C geheizten Blöcken 2 und 3 zugeführt. Bei Block 3 erfolgt eine Zudosierung von 11 kg/h (ca. 30%) Glycerin mit einem Arbeitsdruck von mindestens 10 bar über eine gravimetrische Kolbenpumpe. In den geschlossenen Blöcken 4 bis 6 ist die Temperatur bis auf 140°C erhöht. Bei Block 7 ist ein Unterdruck von 800 mbar angelegt, wobei ca. 6% Wasser abgehen. Die Temperatur ist jetzt wieder auf 110°C zurückgenommen. Bei Block 8 erfolgt eine Zufuhr von 1,4 kg/h (ca. 10%) Kalziumcarbonat. Gegebenenfalls kann an Block 9 1,9 kg/h (ca. 5 bis 8%) Glycerin zudosiert werden. Der Arbeitsdruck beträgt ebenfalls mindestens 10 bar. Falls dieser Anschluss nicht benötigt wird, ist er mit einem Blindstopfen verschlossen. Bei Block 11 ist wiederum ein Unterdruck angelegt, wobei ca. 2 bis 4% Wasser abgehen. Gegebenenfalls genügt aber auch eine nur atmosphärische Belüftung.

Die Temperatur der Schmelze darf an keiner Stelle des Extruders 160°C überschreiten, weil sonst ein thermischer Abbau der Stärke einsetzt. Weiter gilt, dass die thermische Veränderung der Stärke um so geringer ausfällt, je kürzer die Schmelze einer hohen Temperatur ausgesetzt wird. Zwischen Temperatursteuerung und Materialdurchsatz muss daher ein optimales Verhältnis hergestellt werden.

Die vorliegende Erfindung wird anhand der nachstehenden Beispiele weiter erläutert:

## Beispiel 1

Über einen zweiwelligen Extruder (Typ ZSK 30, Werner & Pfleiderer) werden die folgenden Komponenten kontinuierlich dosiert und aufgeschmolzen.

| | |
|---|---|
| Stärke | 7,7 kg/h |
| Lecithin | 0,147 kg/h |
| Glycerin-Monostearat | 0,147 kg/h |
| Glycerin (99,5 Reinheit) | 4,47 kg/h |
| Calciumcarbonat, gefällt | 1.0 kg/h |

Wobei bei einer Schneckendrehzahl von 180 rpm unter folgenden Bedingungen extrudiert wird (siehe Figur 2):

| | |
|---|---|
| Block 1 | 25°C |
| Block 2 und 3 | 100°C |
| Block 4 bis 6 | 140°C |
| Block 7 bis 9 | 110°C |
| Block 10 bis 12 | 110°C |
| Düse: | 110°C |

Bezogen auf die wasserfreie Stärke entspricht dies einem Glyceringehalt von 38,77%. Bezogen auf das wasserfrei Endprodukt resultieren folgende Anteile:

| | |
|---|---|
| Lecithin | 1,11% |
| Glycerin-Monostearat | 1,11% |
| Stärke (wasserfrei) | 55,15% |
| CaCO3 | 7,76% |

Spezifische Energieaufnahme bei der Extrusion: 0,275 kWh/kg

**Beispiel 2**

Über einen zweiwelligen Extruder (Typ ZSK 30,Werner & Pfleiderer) werden die folgenden Komponenten kontinuierlich dosiert und aufgeschmolzen.

| Stärke | 7,7 kg/h |
|---|---|
| Lecithin | 0,147 kg/h |
| Glycerin-Monostearat | 0,147 kg/h |
| Glycerin (99,5% Reinheit) | 4,67 kg/h |

Wobei bei einer Schneckendrehzahl von 260 rpm unter denselben Bedingungen wie in Beispiel 1 extrudiert wird.
Im Block 4 kann alternativ ein Vakuum angelegt werden, um überschüssiges Wasser (aus dem Stärkepulver) abzuziehen. (Z.B. 800 mbar)
Bezogen auf die wasserfreie Stärke entspricht dies einem Glyceringehalt von 39,81 %. Bezogen auf das wasserfreie Endprodukt resultieren folgende Anteile:

| Lecithin | 1,18% |
|---|---|
| Glycerin-Monostearat | 1,18% |
| Stärke (wasserfrei) | 58,81% |

Spezifische Energieaufnahme bei der Extrusion:    0,233 kWh/kg

**Beispiel 3**

Über einen zweiwelligen Extruder (Typ ZSK 30,Werner & Pfleiderer) werden die folgenden Komponenten kontinuierlich dosiert und aufgeschmolzen.

| Stärke | 7,7 kg/h |
|---|---|
| Lecithin | 0,147 kg/h |
| Glycerin-Monostearat | 0,147 kg/h |
| Glycerin (99,5% Reinheit) | 4,47 kg/h |

Wobei bei einer Schneckendrehzahl von 260 rpm unter denselben Bedingungen wie in Beispiel 1 extrudiert wird.
Im Block 4 kann alternativ ein Vakuum angelegt werden, um überschüssiges Wasser (aus dem Stärkepulver) abzuziehen. (Z.B. 800 mbar)
Bezogen auf die wasserfreie Stärke entspricht dies einem Glyceringehalt von 38,77%. Bezogen auf das wasserfreie Endprodukt resultieren folgende Anteile:

| Lecithin | 1,20% |
|---|---|
| Glycerin-Monostearat | 1,20% |
| Stärke (wasserfrei) | 59,79% |

**Beispiel 4**

Über einen zweiwelligen Extruder (Typ ZSK 30,Werner & Pfleiderer) werden die folgenden Komponenten kontinuierlich dosiert und aufgeschmolzen.

| Stärke | 7,7 kg/h |
|---|---|
| Lecithin | 0,147 kg/h |
| Glycerin-Monostearat | 0,147 kg/h |
| Glycerin (99,5% Reinheit) | 4,47 kg/h |
| Calziumcarbonat, gefällt | 1,0 kg/h |

Wobei bei einer Schneckendrehzahl von 260rpm unter denselben Bedingungen wie in Beispiel 1 extrudiert wird.

Im Block 4 kann alternativ ein Vakuum angelegt werden, um überschüssiges Wasser (aus dem Stärkepulver) abzuziehen. (Z.B. 800 mbar)

Bezogen auf die wasserfreie Stärke entspricht dies einem Glyceringehalt von 39,81%. Bezogen auf das wasserfreie Endprodukt resultieren folgende Anteile:

| | |
|---|---|
| Lecithin | 1,09% |
| Glycerin-Monostearat | 1,09% |
| Stärke (wasserfrei) | 54,31% |
| CaCO3 | 7,64% |

Spezifische Energieaufnahme bei der Extrusion: 0,254 kWh/kg

## Beispiel 5

Über einen zweiwelligen Extruder (Typ ZSK 30,Werner & Pfleiderer) werden die folgenden Komponenten kontinuierlich dosiert und aufgeschmolzen.

| | |
|---|---|
| Stärke | 7,7 kg/h |
| Lecithin | 0,147 kg/h |
| Glycerin-Monostearat | 0,147 kg/h |
| Glycerin (99,5% Reinheit) | 4,87 kg/h |
| Calziumcarbonat, gefällt | 1,0 kg/h |

Wobei bei einer Schneckendrehzahl von 260 rpm unter denselben Bedingungen wie in Beispiel 1 extrudiert wird.

Im Block 4 kann alternativ ein Vakuum angelegt werden, um überschüssiges Wasser (aus dem Stärkepulver) abzuziehen. (Z.B. 800mbar)

Bezogen auf die wasserfreie Stärke entspricht dies einem Glyceringehalt von 40,81%. Bezogen auf das wasserfreie Endprodukt resultieren folgende Anteile:

| | |
|---|---|
| Lecithin | 1,08% |
| Glycerin-Monostearat | 1,08% |
| Stärke (wasserfrei) | 53,49% |
| CaCO3 | 7,53% |

Spezifische Energieaufnahme bei der Extrusion: 0,242 kWh/kg

Die so entstehende Masse wird durch die Düse zu einem Strang geformt, diese an der Umgebungsluft abgekühlt und schliesslich granuliert.

Das so erhaltene Granulat wird in einem Ein-Schnecken-Extruder (Chill-Roll-Anlage Kompressionsschnecke (1:3) von Göttfert) bei 130°C und 70rpm aufgeschmolzen und durch eine Schlitzdüse auf ein paar wassergekühlte Walzen mit einer Temperatur < 40°C geführt, um von dort mittels regelbarer Wickleranlage (ebenfalls von Göttfert) mit einer Zwischenschicht aus Polyethylen-Folie aufgewickelt zu werden.

Zwei solcher, identischer Folien-Wickel werden im nächsten Arbeitsschritt kontinuierlich abgewickelt und nach Ablösung der Polyethylen-Zwischenlage auf die zwei Prägewalzen einer Rotary-Die-Anlage geführt.

Tab. 1:

| Die mechanischen Eigenschaften der Stärkefilme mit 41,8% Glycerin in Abhängigkeit des Staudinger-Index [η] | | | | | | |
|---|---|---|---|---|---|---|
| Proben Nr. | $T_B$ °C | $H_2O$ % | [η] ml/g | d mm | $\sigma_m$ MPa | $\varepsilon_b$ % |
| 7 | 110 | 1.77 | 160.5 | 0.72 | 7.0 +/- 0.3 | 107 +/- 6 |
| 6 | 140 | 1.80 | 139.9 | 0.65 | 6.8 +/- 0.4 | 106 +/- 18 |

Tab. 1: (fortgesetzt)

| Die mechanischen Eigenschaften der Stärkefilme mit 41,8% Glycerin in Abhängigkeit des Staudinger-Index $[\eta]$ | | | | | | |
|---|---|---|---|---|---|---|
| Proben Nr. | $T_B$ °C | $H_2O$ % | $[\eta]$ ml/g | d mm | $\sigma_m$ MPa | $\varepsilon_b$ % |
| 5 | 160 | 1.55 | 127.9 | 0.64 | 6.3 +/- 0.4 | 99 +/- 5 |
| 4 | 180 | 1.54 | 115.6 | 0.64 | 6.9 +/- 0.2 | 107+/-9 |
| 3 | 220 | 1.66 | 82.8 | 0.73 | 4.8 +/- 0.4 | 97 +/- 23 |
| 2 | 200 | 1.55 | 59.2 | 0.61 | 4.9 +/- 0.5 | 69 +/- 23 |
| 1 | 235 | 1.30 | 51.5 | 0.87 | 9.0 +/- 0.7 | 22 +/- 24 |

## Patentansprüche

1. Verfahren zum Herstellen eines Stärke enthaltenden Formkörpers, insbesondere einer Weichkapsel mit einteiliger Kapselhülle, **gekennzeichnet durch** folgende Schritte

   a Überführung einer Mischung enthaltend mindestens eine native oder chemisch-modifizierte Stärke mit einem Amylopektingehalt von grösser oder gleich 50 Gew.-% bezogen auf das Gewicht der wasserfreien Stärke, Wasser, und mindestens einen organischen Weichmacher, wobei der Gehalt an organischem Weichmacher in einem Bereich von 37 Gew.-% bis 50 Gew.-% bezogen auf das Gewicht der wasserfreien Stärke liegt, unter Aufschmelzen und Kneten in eine homogenisierte, thermoplastische Schmelzmasse in einer ersten Verarbeitungseinrichtung;
   b gegebenenfalls Herstellen eines lagerfähigen Zwischenproduktes, insbesondere eines Granulates nach Abkühlen der homogenisierten Schmelze und nachfolgendes Einschmelzen des Zwischenproduktes in einer zweiten Verarbeitungseinrichtung;
   c Herstellen wenigstens eines Materialstranges, insbesondere eines extrudierten Films, am Ausgang der ersten oder gegebenenfalls zweiten Verarbeitungseinrichtung,
   d Umformen des Materialstranges zu einem Formkörper in einem kontinuierlichen oder intermittierenden Formverfahren;
   e gegebenenfalls Trocknen des Formkörper,

   wobei die Schritte a) bis c) derart durchgeführt werden, dass in Schritt d) der Wert des Staudinger-Index der den Materialstrang bildenden Masse mindestens 40 ml/g, bevorzugt mindestens 50 ml/g und noch bevorzugter mindestens 60 ml/g beträgt.

2. Verfahren gemäss Anspruch 1, **dadurch gekennzeichnet, dass** die im Schritt a) eingesetzten Mischung zusätzlich ein internes Gleit- und Formtrennmittel enthält, welches ausgewählt ist aus der Gruppe bestehend aus Lecithinen, Mono-, Dioder Triglyceriden der Speisefettsäuren, insbesondere Glycerinmonostearat, Polyglycerinester der Speisefettsäuren, Polyethylenester der Speisefettsäuren, Zuckerester der Speisefettsäuren und Speisefettsäuren, Pyrrolidonen.

3. Verfahren gemäss Anspruch 2, **dadurch gekennzeichnet, dass** die in Schritt a) eingesetzte Mischung Glycerinmonostearat und Lecithin in einem Gewichtsverhältnis von 1:1,5 bevorzugt von 1:1,2 und noch bevorzugter von 1:1 enthält.

4. Verfahren gemäss einem der Ansprüche 1 bis 3, **dadurch gekennzeichnet, dass** der Gehalt an organischem Weichmacher in einem Bereich von 38 Gew.-% bis 45 Gew.-% liegt.

5. Verfahren gemäss einem der Ansprüche 1 bis 4, **dadurch gekennzeichnet, dass** der Weichmacher durch Wasser ersetzt wird, wobei der Ersatz im Verhältnis 2 Teile Weichmacher : 1 Teil Wasser durchgeführt wird und der Mindestgehalt Weichmacher 12 Gew.% bezogen auf das Gewicht der wasserfreien Stärke beträgt.

6. Verfahren gemäss einem der Ansprüche 1 bis 5, **dadurch gekennzeichnet, dass** die Temperatur der Schmelzmasse in den Schritten a) bis c) 160°C, bevorzugt 120°C und noch bevorzugter 90°C nicht übersteigt.

7. Verfahren nach einem der Ansprüche 1 bis 6, **dadurch gekennzeichnet, dass** die durch Kneten eingebrachte Energie in den Schritten a) bis c) 0,3 kWh/kg, bevorzugt 0,2 kWh/kg und noch bevorzugter 0,175 kWh/kg nicht übersteigt.

8. Verfahren nach einem der Ansprüche 1 bis 7, **dadurch gekennzeichnet, dass** mindestens das Aufschmelzen in der ersten Verarbeitungseinrichtung in einem gleichdrehenden Doppel-Schnecken-Extruder erfolgt und dass einzelne Abschnitte des Extruders bezogen auf die Längsrichtung der Schnecken auf unterschiedliche Temperaturen geheizt werden.

9. Verfahren nach einem der Ansprüche 1 bis 8, **dadurch gekennzeichnet, dass** in Schritt c) der Materialstrang als flach geführter Film extrudiert wird, welcher mit Zwischenlagen von antihaftendem Material vorzugsweise als Rollen gelagert und zu einem späteren Zeitpunkt zu Formteilen, insbesondere Kapselhüllen, geformt wird.

10. Verfahren nach einem der Ansprüche 1 bis 9, **dadurch gekennzeichnet, dass** das Umformen in Schritt d) zwei homogene Materialfilme umfasst, welche in einem üblichen Verkapselungsprozess, insbesondere im Rotary-Die-Verfahren, zu Weichkapseln mit einer einteiligen Kapselhülle geformt werden, wobei das Zusammenfügen von Kapselhüllteilen und das Füllen der Kapselhülle in einem Arbeitsschritt erfolgt.

11. Verfahren nach einem der Ansprüche 1 bis 10, **dadurch gekennzeichnet, dass** in Schritt c) ein Film schlauchförmig extrudiert wird, der Schlauch zuerst geschlitzt und als flach geführtes Band in Schritt d) weiterverarbeitet wird.

12. Homogenisierte, Stärke enthaltende Masse, enthaltend mindestens 45 Gew.-% einer amorphen, aus nativer oder chemisch-modifizierter Stärke erhältlichen Stärke mit einem Amylopektingehalt von grösser oder gleich 50 Gew.% bezogen auf das Gewicht der wasserfreien Stärke, Wasser, mindestens einen organischen Weichmacher in einem Anteil im Bereich von 37-50 Gew.-% bezogen auf das Gewicht der wasserfreien Stärke, wobei der Staudinger-Index der homogenisierten Masse mindestens 40 ml/g, bevorzugt mindestens 50 ml/g und noch bevorzugter mindestens 60 ml/g beträgt.

13. Homogenisierte Masse gemäss Anspruch 12, **dadurch gekennzeichnet, dass** die Masse zusätzlich mindestens ein Gleit und Entformmittel enthält, welches ausgewählt ist aus der Gruppe bestehend aus Lecithinen, Mono-, Di- und Triglyceriden von Speisefettsäuren, insbesondere Glycerinmonostearat, Polyglycerinestern der Speisefettsäuren, Polyethylenestern der Speisefettsäuren, Zuckerestem der Speisefettsäuren und Speisefettsäuren.

14. Homogenisierte Masse nach einem der Ansprüche 12 bis 13, **dadurch gekennzeichnet, dass** der Weichmacher ausgewählt wird aus der Gruppe bestehend aus Polyalkoholen, insbesondere Glycerin, organischen Säuren, Hydroxysäuren, Aminen, Säureamiden und Sulfoxiden, Pyrrolidonen.

15. Homogenisierte Masse nach einem der Ansprüche 12 bis 14, **dadurch gekennzeichnet, dass** die Masse Glycerinmonostearat und Lecithin in einem Gewichtsverhältnis von 1:1,5 bevorzugt von 1:1,2 und noch bevorzugter von 1:1 1 enthält.

16. Homogenisierte Masse gemäss einem der Ansprüche 12 bis 15, **dadurch gekennzeichnet, dass** die Masse zusätzlich mindestens einen Zuschlagstoff in einem Gewichtsbereich von 3,5 Gew.-% bis 15 Gew.% bezogen auf das Gesamtgewicht der Masse enthält, bevorzugt von 5 Gew.% bis 8 Gew.%, wobei der Zuschlagstoff ausgewählt ist aus der Gruppe bestehend aus Carbonaten und/oder Hydrogencarbonaten der Alkali- und/oder Erdalkaliionen, bevorzugt Kalziumcarbonat, Amylasen, weiteren Zerfallshilfen, Farbstoffen, Konservierungsmitteln, Antioxidantien, physikalisch und/oder chemisch modifizierten Biopolymeren und pflanzlichen Polypeptiden.

17. Formkörper, insbesondere Weichkapselhülle, hergestellt aus einer Masse gemäss einem der Ansprüche 12 bis 16 und/oder einem Verfahren gemäss einem der Ansprüche 1 bis 11.

18. Formkörper, insbesondere Weichkapselhülle, gemäss Anspruch 17, **dadurch gekennzeichnet, dass** der Formkörper eine Bruchdehnung von mindestens 100%, bevorzugt mindestens 160% und noch bevorzugter mindestens 240% bei 25°C und 60% relativer Luftfeuchtigkeit aufweist.

**19.** Formkörper, insbesondere Weichkapselhülle, nach einem der Ansprüche 17 bis 18, **dadurch gekennzeichnet, dass** der Formkörper bei 25°C und 60% relativer Luftfeuchtigkeit eine Festigkeit $\sigma_m$ von mindestens 2MPa, bevorzugt eine Festigkeit im Bereich von 3,5Mpa bis 8MPa und noch bevorzugter von 4MPa bis 6,5MPa aufweist.

**20.** Formkörper nach einem der Ansprüche 17 bis 19, **dadurch gekennzeichnet, dass** der Formkörper eine Weichkapsel ist und dass die Kapselhülle eine Dicke im Bereich zwischen 0,1 und 2 mm bevorzugt zwischen 0,2 und 0,6 mm aufweist.

**21.** Formkörper, insbesondere Weichkapselhülle, gemäss einem der Ansprüche 17 bis 20, **dadurch gekennzeichnet, dass** der Formkörper aus einem mehrlagigen Film besteht und dass mindestens zwei der Filme eine unterschiedliche chemische Zusammensetzung aufweisen.

**22.** Vorrichtung zum Herstellen einer Weichkapsel, bestehend aus einer einteiligen Kapselhülle und einem Kapselinhalt, aus einer Masse nach einem der Ansprüche 12 bis 15 in einem Verfahren nach einem der Ansprüche 1 bis 11, wobei die Vorrichtung umfasst:

a) je einem neben einer Füll- und Formstation angeordneten Extruder zur Herstellung mindestens zwei bahnförmiger Filme,
b) eine Füll- und Formstation zur Formung einer Kapselhülle in einem Formverfahren aus mindestens zwei bahnförmigen Filmen und zur Befüllung mit einem Kapselinhalt

**dadurch gekennzeichnet, dass** Extruder und Form- und Füllstation so nebeneinander angeordnet sind, dass die bahnförmigen Filme aus den Extrudem unmittelbar in die Füll- und Formstation zur Herstellung der Weichkapsel einführbar sind.

## Claims

**1.** Process for the production of a starch-containing shaped body, in particular a soft capsule with single-part capsule shell, **characterized by** the following steps

a conversion of a mixture comprising at least one native or chemically modified starch with an amylopectin content of greater than or equal to 50% by weight, based on the weight of the anhydrous starch, water, and at least one organic plasticizer, where the content of organic plasticizer is in the range from 37% by weight to 50% by weight, based on the weight of the anhydrous starch, with melting and kneading, into a homogenized, thermoplastic molten composition in a first processing apparatus;
b if appropriate, preparation of a storable intermediate product, in particular of granules after cooling of the homogenized melt and subsequent melting of the intermediate product in a second processing apparatus;
c production of at least one extrudate, in particular of an extruded film, at the exit from the first or, if appropriate, second processing apparatus,
d forming of the extrudate to give a shaped body in a continuous or intermittent forming process;
e if appropriate, drying of the shaped body,

where the steps a) to c) are carried out in such a way that in step d) the value of the Staudinger index of the composition forming the extrudate is at least 40 ml/g, preferably at least 50 ml/g and more preferably at least 60 ml/g.

**2.** Process according to Claim 1, **characterized in that** the mixture used in step a) also comprises an internal lubricant and internal mould-release agent, which has been selected from the group consisting of lecithins, mono-, di- or triglycerides of the edible fatty acids, in particular glycerol monostearate, polyglycerol esters of the edible fatty acids, polyethylene esters of the edible fatty acids, sugar esters of the edible fatty acids and edible fatty acids, pyrrolidones.

**3.** Process according to Claim 2, **characterized in that** the mixture used in step a) comprises glycerol monostearate and lecithin in a ratio by weight of 1:1.5, preferably of 1:1.2 and more preferably of 1:1.

**4.** Process according to any of Claims 1 to 3, **characterized in that** the content of organic plasticizer is in the range from 38% by weight to 45% by weight.

# EP 1 103 254 B1

5. Process according to any of Claims 1 to 4, **characterized in that** the plasticizer is replaced by water, the replacement being carried out in the ratio of 2 parts of plasticizer : 1 part of water and the minimum content of plasticizer being 12% by weight, based on the weight of the anhydrous starch.

6. Process according to any of Claims 1 to 5, **characterized in that** the temperature of the molten composition in steps a) to c) does not exceed 160°C, preferably 120°C and more preferably 90°C.

7. Process according to any of Claims 1 to 6, **characterized in that** the energy introduced via kneading in steps a) to c) does not exceed 0.3 kWh/kg, preferably 0.2 kWh/kg and more preferably 0.175 kWh/kg.

8. Process according to any of Claims 1 to 7, **characterized in that** at least the melting in the first processing apparatus takes place in a corotating twin-screw extruder, and that individual sections of the extruder, based on the longitudinal direction of the screws, are heated to different temperatures.

9. Process according to any of Claims 1 to 8, **characterized in that** in step c) the extrudate is extruded in the form of a film conducted so as to be flat, and preferably stored in the form of rolls with interlays of release material, and at a later juncture formed to give shaped bodys, in particular capsule shells.

10. Process according to any of Claims 1 to 9, **characterized in that** the forming in step d) encompasses two homogeneous material films which are subjected to forming in a conventional encapsulation process, in particular in the rotary die process, to give soft capsules with a single-part capsule shell, where the joining of capsule shell parts and the filling of the capsule shell takes place in one operation.

11. Process according to any of Claims 1 to 10, **characterized in that** in step c) a film is extruded in the form of a tube, and the tube is first slit and is further processed in step d) in the form of a strip conducted so as to be flat.

12. Homogenized, starch-containing composition, comprising at least 45% by weight of an amorphous starch obtainable from native or from chemically modified starch and having an amylopectin content greater than or equal to 50% by weight, based on the weight of the anhydrous starch, water, a proportion in the range from 37 to 50% by weight, based on the weight of the anhydrous starch, of at least one organic plasticizer, where the Staudinger index of the homogenized composition is at least 40 ml/g, preferably at least 50 ml/g and more preferably at least 60 ml/g.

13. Homogenized composition according to Claim 12, **characterized in that** the composition also comprises at least one lubricant and mould-release agent selected from the group consisting of lecithins, mono-, di- or triglycerides of the edible fatty acids, in particular glycerol monostearate, polyglycerol esters of the edible fatty acids, polyethylene esters of the edible fatty acids, sugar esters of the edible fatty acids and edible fatty acids.

14. Homogenized composition according to any of Claims 12 to 13, **characterized in that** the plasticizer is selected from the group consisting of polyalcohols, in particular glycerol, organic acids, hydroxy acids, amines, amides and sulphoxides, pyrrolidones.

15. Homogenized composition according to any of Claims 12 to 14, **characterized in that** the composition comprises glycerol monostearate and lecithin in a ratio by weight of 1:1.5, preferably 1:1.2 and more preferably 1:1.

16. Homogenized composition according to any of Claims 12 to 15, **characterized in that** the composition also comprises at least one additive in a range by weight of from 3.5% by weight to 15% by weight, based on the total weight of the composition, preferably from 5% by weight to 8% by weight, where the additive is one selected from the group consisting of carbonates and/or hydrogencarbonates of the ions of alkali metals and/or of alkaline earth metals, preferably calcium carbonate, amylases, other disintegrants, dyes, preservatives, antioxidants, physically and/or chemically modified biopolymers and vegetable polypeptides.

17. Shaped body, in particular soft capsule shell, produced from a composition according to any of Claims 12 to 16 and/or from a process according to any of Claims 1 to 11.

18. Shaped body, in particular soft capsule shell, according to Claim 17, **characterized in that** the shaped body has a tensile strain at break of at least 100%, preferably at least 160% and more preferably at least 240% at 25°C and 60% relative humidity.

15

**19.** Shaped body, in particular soft capsule shell, according to any of Claims 17 to 18, **characterized in that** the shaped body has a strength $\sigma_m$ of at least 2 MPa at 25°C and 60% relative humidity, preferably a strength in the range from 3.5 MPa to 8 MPa and more preferably of from 4 MPa to 6.5 MPa.

**20.** Shaped body according to any of Claims 17 to 19, **characterized in that** the shaped body is a soft capsule and that the capsule shell has a thickness in the range from 0.1 to 2 mm, preferably from 0.2 to 0.6 mm.

**21.** Shaped body, in particular soft capsule shell, according to any of Claims 17 to 20, **characterized in that** the shaped body is composed of a multilayer film, and that at least two of the films have a different chemical constitution.

**22.** Device for the production of a soft capsule, composed of a single-part capsule shell and of capsule content, composed of a composition according to any of Claims 12 to 15 in a process according to any of Claims 1 to 11, where the device encompasses:

> a) alongside each filling and forming unit, an extruder arranged for the production of at least two films in the form of webs,
> b) a filling and forming unit for the forming of a capsule shell in a forming process from at least two films in the form of webs, and for filling with capsule content,

> **characterized in that** the extruder and the forming and filling unit have been arranged alongside one another in such a way that the films in the form of webs from the extruders can be passed immediately into the filling and forming unit for the production of the soft capsule.

**Revendications**

**1.** Procédé de fabrication d'un objet façonné contenant de l'amidon, en particulier d'une capsule molle à enveloppe en une seule pièce, **caractérisé par** les étapes suivantes :

> a. dans un premier dispositif de mise en oeuvre, la transformation d'un mélange contenant au moins un amidon natif ou chimiquement modifié, ayant une teneur en amylopectine supérieure ou égale à 50 % en poids par rapport au poids de l'amidon anhydre, de l'eau, et au moins un plastifiant organique, la teneur en le plastifiant organique étant comprise dans une plage de 37 à 50 % en poids par rapport au poids de l'amidon anhydre, par fusion et malaxage, en une masse fondue thermoplastique homogénéisée ;
> b. éventuellement, la préparation d'un produit intermédiaire stable au stockage, en particulier d'un granulat après refroidissement de la masse fondue homogénéisée, puis la fusion du produit intermédiaire dans un deuxième dispositif de mise en oeuvre ;
> c. la fabrication d'au moins un cordon de matière, en particulier d'un film extrudé, en sortie du premier ou éventuellement du deuxième dispositif de mise en oeuvre ;
> d. le formage du cordon de matière, pour obtenir un objet façonné dans le cadre d'un procédé de formage continu ou intermittent ;
> e. éventuellement, le séchage de l'objet façonné, les étapes a) à c) étant mises en oeuvre de telle sorte que, dans l'étape d), la valeur de l'indice de Staudinger de la masse formant le cordon de matière soit d'au moins 40 ml/g, de préférence d'au moins 50 ml/g et d'une manière encore plus préférée d'au moins 60 ml/g.

**2.** Procédé selon la revendication 1, **caractérisé en ce que** le mélange utilisé dans l'étape a) contient en outre un agent lubrifiant et de démoulage interne, qui est choisi dans le groupe consistant en les lécithines, les mono-, di- ou triglycérides des acides gras alimentaires, en particulier le monostéarate de glycérol, les polyglycérol esters des acides gras alimentaires, les polyéthylène esters des acides gras alimentaires, les esters de sucre des acides gras alimentaires et les acides gras alimentaires, les pyrrolidones.

**3.** Procédé selon la revendication 2, **caractérisé en ce que** le mélange utilisé dans l'étape a) contient du monostéarate de glycérol et de la lécithine selon un rapport en poids de 1:1,5, de préférence de 1:1,2 et d'une manière encore plus préférée de 1:1.

**4.** Procédé selon l'une des revendications 1 à 3, **caractérisé en ce que** la teneur en le plastifiant organique est comprise dans une plage de 38 à 45 % en poids.

5. Procédé selon l'une des revendications 1 à 4, **caractérisé en ce que** le plastifiant est remplacé par de l'eau, le remplacement étant réalisé selon un rapport de 2 parties de plastifiant pour 1 partie d'eau, la teneur minimale en le plastifiant étant de 12 % en poids par rapport au poids de l'amidon anhydre.

6. Procédé selon l'une des revendications 1 à 5, **caractérisé en ce que** la température de la masse fondue dans les étapes a) à c) ne dépasse pas 160°C, de préférence pas 120°C et d'une manière encore plus préférée pas 90°C.

7. Procédé selon l'une des revendications 1 à 6, **caractérisé en ce que** l'énergie apportée par le malaxage dans les étapes a) à c) ne dépasse pas 0,3 kWh/kg, de préférence pas 0,2 kWh/kg et d'une manière encore plus préférée pas 0,175 kWh/kg.

8. Procédé selon l'une des revendications 1 à 7, **caractérisé en ce qu'**au moins la fusion dans le premier dispositif de mise en oeuvre est réalisée dans une extrudeuse à deux vis tournant dans le même sens, et que des sections individuelles de l'extrudeuse, s'étendant dans le sens longitudinal des vis, sont chauffées à des températures différentes.

9. Procédé selon l'une des revendications 1 à 8, **caractérisé en ce que**, dans l'étape c), le cordon de matière est extrudé sous forme d'un film guidé à plat, qui est stocké sous forme de bobines, avec des couches intermédiaires d'un matériau antiadhésif, et ultérieurement est soumis à un formage pour donner des objets façonnés, en particulier des enveloppes de capsule.

10. Procédé selon l'une des revendications 1 à 9, **caractérisé en ce que** le formage dans l'étape d) englobe deux films de matière homogène, qui sont formés dans un procédé usuel d'encapsulage, en particulier par un procédé à découpeuse rotative, pour donner des capsules molles ayant une enveloppe en une seule pièce, l'assemblage des parties de l'enveloppe et le remplissage de l'enveloppe des capsules s'effectuant dans une seule étape.

11. Procédé selon l'une des revendications 1 à 10, **caractérisé en ce que**, dans l'étape c), un film est extrudé sous forme d'un tube souple, ce tube souple est d'abord fendu, puis est soumis à une post-transformation dans l'étape d) sous forme d'une bande guidée à plat.

12. Masse homogénéisée contenant de l'amidon, contenant au moins 45 % en poids d'un amidon amorphe, pouvant être obtenu à partir d'amidon natif ou chimiquement modifié, ayant une teneur en amylopectine supérieure ou égale à 50 % en poids par rapport au poids de l'amidon anhydre, de l'eau, au moins un plastifiant organique en une quantité comprise dans la plage de 37 à 50 % en poids par rapport au poids de l'amidon anhydre, l'indice de Staudinger de la masse homogénéisée étant d'au moins 40 ml/g, de préférence d'au moins 50 ml/g et d'une manière encore plus préférée d'au moins 60 ml/g.

13. Masse homogénéisée selon la revendication 12, **caractérisée en ce que** la masse contient en outre au moins un agent lubrifiant et de démoulage, qui est choisi dans le groupe consistant en les lécithines, les mono-, di- et triglycérides d'acides gras alimentaires, en particulier le monostéarate de glycérol, les polyglycérol esters des acides gras alimentaires, les polyéthylène esters des acides gras alimentaires, les esters de sucre des acides gras alimentaires et les acides gras alimentaires.

14. Masse homogénéisée selon l'une des revendications 12 et 13, **caractérisée en ce que** le plastifiant est choisi dans le groupe consistant en les polyalcools, en particulier le glycérol, les acides organiques, les hydroxyacides, les amines, les amides d'acides et les sulfoxydes, les pyrrolidones.

15. Masse homogénéisée selon l'une des revendications 12 à 14, **caractérisée en ce que** la masse contient du monostéarate de glycérol et de la lécithine selon un rapport en poids de 1:1,5, de préférence de 1:1,2 et d'une manière encore plus préférée de 1:1.

16. Masse homogénéisée selon l'une des revendications 12 à 15, **caractérisée en ce que** la masse contient en outre au moins un adjuvant en une quantité pondérale de 3,5 % en poids à 15 % en poids par rapport au poids total de la masse, de préférence de 5 à 8 % en poids, l'adjuvant étant choisi dans lé groupe consistant en les carbonates et/ou hydrogénocarbonates des ions de métaux alcalins et/ou alcalino-terreux, de préférence le carbonate de calcium, les amylases, d'autres auxiliaires de désintégration, les colorants, les conservateurs, les antioxydants, les biopolymères à modification physique et/ou chimique, et les polypeptides végétaux.

**17.** Objet façonné, en particulier enveloppe pour capsules molles, fabriqué à partir d'une masse selon l'une des revendications 12 à 16 et/ou par un procédé selon l'une des revendications 1 à 11.

**18.** Objet façonné, en particulier enveloppe pour capsules molles, selon la revendication 17, **caractérisé en ce qu'**il présente un allongement à la rupture d'au moins 100 %, de préférence d'au moins 160 % et d'une manière encore plus préférée d'au moins 240 % à 25°C et pour une humidité relative de l'air de 60 %.

**19.** Objet façonné, en particulier enveloppe pour capsules molles, selon l'une des revendications 17 et 18, **caractérisé en ce qu'**il présente à 25°C et pour une humidité relative de l'air de 60 % une résistance mécanique $\sigma_m$ d'au moins 2 MPa, de préférence une résistance mécanique comprise dans la plage de 3,5 à 8 MPa et d'une manière encore plus préférée de 4 à 6,5 MPa.

**20.** Objet façonné selon l'une des revendications 17 à 19, **caractérisé en ce qu'**il est une capsule molle, et que l'enveloppe de la capsule présente une épaisseur comprise dans la plage située entre 0,1 et 2 mm et de préférence entre 0,2 et 0,6 mm.

**21.** Objet façonné, en particulier enveloppe pour capsules molles, selon l'une des revendications 17 à 20, **caractérisé en ce qu'**il est constitué d'un film multicouche, et qu'au moins deux des films ont des compositions chimiques différentes.

**22.** Appareillage pour fabriquer une capsule molle, constituée d'une enveloppe de capsule en une pièce et d'un contenu de capsule, à partir d'une masse selon l'une des revendications 12 à 15 dans un procédé selon l'une des revendications 1 à 11, l'appareillage comprenant :

a) des extrudeuses disposées à côté d'un poste de remplissage et de formage, chacune étant destinée à la fabrication d'au moins deux films en forme de bande,
b) un poste de remplissage et de formage pour former une enveloppe de capsule dans un procédé de formage à partir d'au moins deux films en forme de bande, et pour remplir l'enveloppe d'un contenu de capsule,

**caractérisé en ce que** les extrudeuses et le poste de formage et de remplissage sont disposées les uns à côté des autres de telle sorte que les films en forme de bande puissent, en sortant des extrudeuses, être immédiatement introduits dans la station de remplissage et de formage pour fabriquer les capsules molles.

Fig. 1

Fig. 2.

EP 1 103 254 B1

Fig.3

Fig.4